Europäisches Patentamt

European Patent Office

Office européen des brevets

(11) Veröffentlichungsnummer: **0 201 703**

**A2**

(12) # EUROPÄISCHE PATENTANMELDUNG

(21) Anmeldenummer: 86104347.9

(22) Anmeldetag: 29.03.86

(51) Int. Cl.⁴: **A 61 B 19/00**
**A 61 B 17/22**

(30) Priorität: 11.05.85 DE 3517020

(43) Veröffentlichungstag der Anmeldung:
20.11.86 Patentblatt 86/47

(84) Benannte Vertragsstaaten:
CH FR GB IT LI NL SE

(71) Anmelder: DORNIER SYSTEM GmbH
Postfach 1360
D-7990 Friedrichshafen(DE)

(72) Erfinder: Hepp, Wolfgang, Dr.
Hardtstrasse 6
D-7997 Immenstaad(DE)

(72) Erfinder: Heine, Gerold, Dr.
Reismühlenweg 7
D-7772 Uhldingen-Mühlhofen 1(DE)

(74) Vertreter: Landsmann, Ralf, Dipl.-Ing.
Kleeweg 3
D-7990 Friedrichshafen 1(DE)

(54) Berührungslose Bearbeitung, insbesondere Zerstörung von künstlich hergestellten Werkstoffen, Bauteilen und Strukturteilen.

(57) Mit Hilfe von Stoßwellen wird Knochenzement, der Microspheres aufweist, zerstört.

1000 µm

EP 0 201 703 A2

DORNIER SYSTEM GMBH — 1 —

7990 Friedrichshafen

Reg. S 513 EU

Berührungslose Bearbeitung, insbesondere Zerstörung von künstlich hergestellten Werkstoffen, Bauteilen und Strukturteilen.

Die Erfindung betrifft ein Verfahren, Werkstoffe und Anwendungen von berührungslos zerstörbaren Werkstoffen, Bauteilen und Strukturteilen.

Es ist bekannt, Nierensteine, die sich im Körper eines Patienten befinden, mittels Stoßwellen berührungsfrei zu zerstören. Diese Fremdkörper im Gewebe eines Lebewesens weisen gegenüber dem Gewebe einen unterschiedlichen Schallwellenwiderstand auf, so daß Abplatzeffekte bei Einwirkung von Stoßwellen auftreten und ihre Sprödigkeit führt dazu, daß die Steine zu abgangsfähigen Teilen zertrümmert werden können. Die Festigkeitseigenschaften solcher Nierensteine sind sehr unterschiedlich. Im menschlichen Körper verursachen die Steine häufig heftige Schmerzen, so daß das Bemühen von Medizin und Technik auf die Beseitigung der Steine gerichtet ist.

Festigkeitslehre und Werkstoffprüfung kennen die zerstörende Werkstoffprüfung durch mechanische Einwirkungen und auch die Zerstörung (Trennen) von Bauteilen durch Wärmeeinwirkung z. B. mittels Gasflamme oder Laser.

Der Erfindung liegt die Aufgabe zugrunde, ein Verfahren zur berührungsfreien Bearbeitung und Zerstörung von Werkstoffen und Bauteilen zu schaffen, die definierte Festigkeitseigenschaften aufweisen.

Insbesondere liegt der Erfindung die Aufgabe zugrunde, Gegenstände und Werkstücke zu zerstören, die sich an unzugänglichem Ort befinden, ohne daß die Umgebung der Gegenstände und Werkstücke bleibend geschädigt wird.

Die Lösung dieser Aufgaben ist Gegenstand des Hauptanspruchs und der Unteransprüche.

Die Erfindung wird nachfolgend bezüglich ihres physikalischen Hintergrunds und anhand von Ausführungsbeispielen näher beschrieben.

Physikalischer Hintergrund

Stoßwellen können zur Zertrümmerung von spröden Materialien
(z.B. Konkrementen in biologischem Gewebe) eingesetzt werden, wenn diese Materialien einen deutlichen akustischen
Impedanzsprung zur Umgebung aufweisen. Der Angriffspunkt
für die Stoßwelle ist auf die Grenzflächen beschränkt. Die
Zertrümmerungswirkung hängt dabei im wesentlichen von der
Anstiegszeit des Druckpulses ab, die Trümmergröße dagegen
von der zeitlichen (räumlichen) Ausdehnung des Druckpulses.
Partikel, die klein gegenüber der Ausdehnung des Druckpulses sind ($\emptyset < 300 \mu$m), erfahren keine irreversiblen Veränderungen.

Eine Stoßwellenwirkung an Mikropartikeln läßt sich dann erzeugen, wenn an oder vorteilhaft in diesen Partikeln Gaseinschlüsse vorhanden sind, die sich unter Einfluß der
Stoßwelle ausdehnen, ihre Schale (Umhüllung) aufreißen,
falls die entstehenden Zugspannungen ausreichen, und anschließend kollabieren mit zusätzlich zerstörenden Wirkungen wie Micro-Jet-Bildung bzw. Micro-Streaming. Die Energiekonzentration in der Mikroblase ist dabei ca. $10^3$
größer als in einem gleich großen Mikropartikel ohne Gaseinschluß. Wichtig ist, daß das Frequenzspektrum der Stoßwelle die Resonanzfrequenz(en) der Mikroblasen einschließt.

( Resonanzfrequenz $f_R \cong \dfrac{1}{2\pi R_O} \left( \dfrac{3KP_O}{\rho} \right)^{1/2}$

$2R_O$ = Durchmesser Mikroblase (Mikrosphere)

$K$ = $1 \leq K \leq 1.4$ Adiabatenexponent

$P_O$ = statischer Druck des Umgebungsmediums

$\rho$ = Dichte des Umgebungsmediums)

Die Energiekonzentration in der Mikroblase hängt stark vom einfallenden Schallfeld und von $R_O$ ab und dürfte bei $2\,R_O$ = 1 $\mu$m im Bereich von einigen 10 millierg liegen, d.h. die Energiedichte besträgt etwa 10 Ws/cm$^3$. Berücksichtigt man die Energiekonzentration beim Blasenkollaps, ist die anzusetzende Energiedichte entsprechend höher.

Die Ausnutzung des beschriebenen Effektes erfordert die Existenz von Mikroblasen im Werkstoff. Da dies i.A. nicht der Fall ist, müssen handhabbare Mikroblasen in den Werkstoff eingebracht werden. Dies können z.B. Microspheres mit einem $\emptyset$ < 300 $\mu$m sein, die z. T. oder gänzlich mit Gas gefüllt sind und eine Hülle aus einem Festkörper (Glas etc.) oder auch aus organischem Material (Sacharin etc.) haben.

Beispiel 1:

Lokale Freisetzung von flüssigen oder festen Stoffen, z.B. Medikamenten, in biologischen Medien.

Die gezielte lokale und nichtinvasive Freisetzung von Medikamenten in biologischen Systemen ist bisher nicht gelöst. Eine potentielle Anwendung ist die lokale und damit organschonende Chemotherapie bei Krebserkrankungen.

Die Medikamente werden in Microspheren (Ø 1 µm bis 300 µm) aus einem biologisch verträglichen und abbaubaren Material, z.B. Sacharin, eingeschlossen. Beim Herstellungsprozeß wird dafür gesorgt, daß ungelöstes Gas (z.B. Luft, $O_2$) in Form von Mikrokeimen eingeschlossen wird. Die Mikrospheres können via Blutbahn oder auch durch direkte Injektion in die interessierende Körperregion gebracht werden und dann durch fokussierte Stoßwellen zertrümmert werden.

Versuche an gasgefüllten Microspheren (Ø 20 um) haben gezeigt, daß pro Stoßwelle ($p_{max} \approx$ 1000 bar) mehrere $10^3$ Spheres zertrümmert werden können. Das Volumen, in dem Microspheres zertrümmert werden können, kann über schallfokussierende Bauteile (Reflektoren, Linsen) im Bereich von einigen $mm^3$ bis einige $cm^3$ variiert werden.

Beispiel 2

betrifft einen Knochenzement, wie er zum Verbinden von Endoprothesen mit Knochenmaterial verwendet wird. Solche Zemente werden insbesondere bei der Implantation von künstlichem Knie oder Hüftgelenk verwendet.

Infolge der Lockerung von Hüftgelenkprothesen sind heute bereits mehr als 5o % aller Hüftgelenkoperationen Reoperationen. Vor dem Einbau einer neuen Endoprothese müssen die Zementreste des Knochenzementbettes entfernt werden.

Bekannt sind Knochenzemente, die nach ihrer Aushärtung durch mechanische Verfahren, wie Bohren oder Fräsen, entfernt werden. Dies erfordert eine erhebliche Krafteinwirkung auf den Knochen. Dieses Vorgehen führt außerdem zu Verletzungen der noch vorhandenen Knochenstruktur. Infolge der unregelmäßigen geometrischen Form ist es schwierig, die Reste vollständig zu entfernen.

Bekannt sind auch poröse Knochenzemente mit offener, durchgehender Porenstruktur (wie z.B. aus der DE-AS 22 42 867). Diese Zemente können auch nur mit mechanischen Methoden, wie Bohren oder Fräsen entfernt werden. Für andere Zerstörungsmöglichkeiten sind die Porenvolumen zu klein.

Aufgabe des Beispiels ist es, einen Knochenzement zu schaffen, der vollständig und mit möglichst geringer Belastung für die noch vorhandene Knochenstruktur entfernt werden kann.

Gelöst wird diese Aufgabe von einem Knochenzement mit mehr als 1 % gasgefüllten Hohlräumen im genannten Größenbereich.

Der erfindungsgemäße Knochenzement kann durch mechanische Stoßwellen zerstört und vollständig beseitigt werden. Die auftretenden Stoßwellen, wobei bevorzugt fokussierte Stoßwellen benutzt werden, bringen die gasgefüllten Hohlräume zur Explosion oder Implosion und zerstören so den umliegenden Knochenzement.

Die Stoßwellen können durch direkte Berührung des Knochens, des Zements oder der Prothese mit einem Schwinger oder berührungsfrei über Luft, Ankoppelkissen oder Gewebe eingeleitet werden. Ein Gerät zur Erzeugung und Einleitung von Stoßwellen im Körper von Lebewesen ist z.B. aus der DE-PS 23 51 247 bekannt.

Die Erfindung erlaubt die einfache Entfernung von gelockerten Implantaten durch eine schnelle, restlose Zementzerkleinerung, die das bestehende Knochengewebe nicht verletzt. Die Stoßwellen zerkleinern oder zerkrümmeln den Zement, ohne bedeutende Kräfte auf den Knochen abzugeben, wie es bei mechanischen Entfernungen bisher der Fall war. Die Einleitung von Stoßwellen in den menschlichen Körper ist eine inzwischen erprobte Technik. Gewebe, das von Stoßwellen durchlaufen wird, wird dabei nicht geschädigt.

Die Abmessungen und die Verteilung der Hohlräume sind so an die Wellenlänge der verwendeten Stoßwellen angepaßt, daß die Einwirkung der Stoßwellen auf die Hohlräume dem Resonanzfall nahe kommt.

Die Herstellung des Zements erfolgt durch aus der Schaumkunststofftechnik an sich bekannte Verfahren des Aufschäumens, wie z.B. durch ein Schaumeinschlagverfahren oder durch Verschäumen mit Treibmitteln.

Die Gaseinschlüsse können auch durch einen chemischen Prozeß während des Aushärtungsprozesses aus geeignet ausgewähltem Ausgangsstoff mit entsprechend feiner Verteilung erzeugt werden. Die beim Aushärtungsprozeß entstehende Wärme kann die Reaktionsenergie für diesen Prozeß liefern. Es können aber auch direkt Schaumblasen untergeschlagen oder untergemischt werden oder kleine Kügelchen (Hohlkügelchen, Mikrospheres) entsprechender Größe in eine der Zementkompenenten vor dem Vermischen und Aushärten eingebracht werden. Bevorzugt können hier billige Hohlkörper, wie Mikrohohlglaskügelchen verwendet werden.

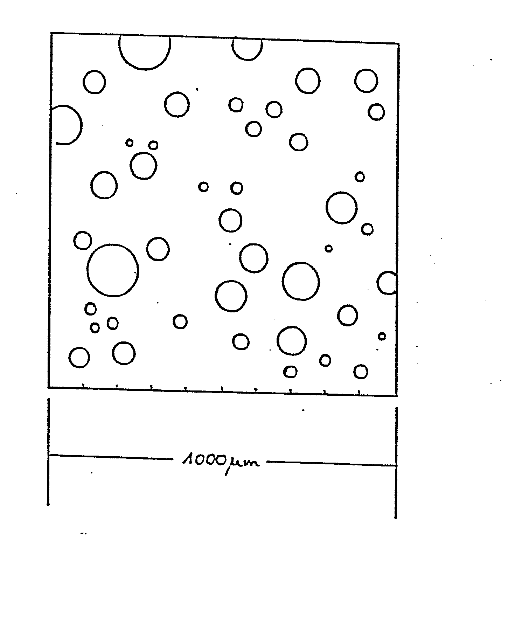

Die einzige Figur zeigt die prinzipielle Verteilung von Mikrospheres in einem Basismaterial, wobei die Größe der Mikrospheres 20 µm bis 120 µm beträgt.

24.2.86
PaL/mr

DORNIER SYSTEM GMBH      — 1 —

7990 Friedrichshafen


S 513 EU



Patentansprüche


1. Verfahren zur berührungslosen Bearbeitung, insbesondere Zerstörung oder teilweiser Zerstörung von künstlich erzeugten Werkstoffen und daraus hergestellten Bauteilen und Strukturteilen, die definierte Festigkeitseigenschaften aufweisen, dadurch gekennzeichnet, dass eine Stoßwelle oder eine Folge einzelner Stoßwellen mittels eines Koppelmediums zum Werkstoff gelangt und beim Durchlaufen des Werkstoffs auf Inhomogenitäten mit unterschiedlichem Schallwellenwiderstand trifft und das inhomogene Material unter Einwirkung der Stoßwelle zerbirst oder zerbröselt.


2. Verfahren nach Anpruch 1, dadurch gekennzeichnet, daß eine fokussierte Stoßwelle oder eine Folge fokussierter Stoßwellen auf das inhomogene Material trifft.


3. Verfahren nach Ansprüchen 1, 2, dadurch gekennzeichnet, daß sich der zu bearbeitende oder zu zerstörende Werkstoff an nicht oder nur schwer zugänglicher Stelle von Lebewesen, Pflanzen, Bauwerken, Maschinen, Geräten oder dergl. befindet.

4. Mittels Stoßwellen zerstörbarer Werkstoff für Verfahren nach Ansprüchen 1 - 4, dadurch gekennzeichnet, daß der Werkstoff Mikropartikeln mit Gaseinschlüssen (Mikrospheres) aufweist.

5. Mittels Stoßwellen zerstörbarer Werkstoff für Verfahren nach Ansprüchen 1 - 4, dadurch gekennzeichnet, daß der Werkstoff Mikroblasen aufweist.

6. Werkstoff nach Ansprüchen 4 - 6, dadurch gekennzeichnet, daß das Gaseinschlüsse enthaltende Material gesintertes Metallpulver, Zement, Gießharz oder Klebematerial oder biologisches Material ist.

7. Verwendung von Werkstoff nach Ansprüchen 4 - 6 als Knochenzement.

8. Verwendung von Werkstoffen nach den Ansprüchen 4 - 6 als Standardmaterial zur Zertrümmerungskontrolle von Stoßwellenapparaturen.

9. Verwendung von Werkstoffen nach Ansprüchen 4 - 6 zur lokalen Stofffreisetzung in biologischen Medien zur therapeutischen Anwendung.

24.2.86 -
PaL/mr

1/1

1000 μm